(19) Europäisches Patentamt
European Patent Office
Office européen des brevets

(11) Numéro de publication: 0 161 979
B1

# (12) FASCICULE DE BREVET EUROPÉEN

(45) Date de publication du fascicule du brevet: 14.01.87

(51) Int. Cl.⁴: C 07 C 2/40, C 07 C 2/46, C 07 C 2/52

(21) Numéro de dépôt: 85400817.4

(22) Date de dépôt: 26.04.85

(54) Procédé de dimérisation d'un diène conjugué.

(30) Priorité: 10.05.84 FR 8407189
13.03.85 FR 8503671

(43) Date de publication de la demande: 21.11.85 Bulletin 85/47

(45) Mention de la délivrance du brevet: 14.01.87 Bulletin 87/3

(84) Etats contractants désignés: AT BE CH DE FR GB IT LI LU NL SE

(56) Documents cité: US-A-3 284 529

(73) Titulaire: Société Chimique des Charbonnages S.A., Tour Aurore Place des Reflets, F-92080 Paris La Défense 2 Cédex 5 (FR)

(72) Inventeur: Mortreux, André, 17, rue Gambetta, F-59510 Hem (FR)
Inventeur: Petit, Françis, 27 Avenue de Flandres, F-59290 Wasquehal (FR)
Inventeur: Denis, Philippe, 46/34 rue des Victoires, F-59650 Villeneuve D'Ascq (FR)
Inventeur: Buono, Gérard, 171 Avenue des Caillols, Bât.A, F-13012 Marseille (FR)
Inventeur: Peiffer, Gilbert, 9 Avenue Paul Carrère, F-13012 Marseille (FR)

(74) Mandataire: Dubost, Thierry Pierre, SOCIETE CHIMIQUE DES CHARBONNAGES Service Propriété Industrielle B.P. 57, F-62670 Mazingarbe (FR)

EP 0 161 979 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un procédé de dimérisation de dienes conjugués.

Le brevet américain n° 3 284 529 décrit la production de dimères linéaires de composés aliphatiques dioléfiniques conjugués en mettant ces composés à une température de 70° à 160°C en présence d'un catalyseur de nickel zérovalent dérivé du nickel carbonyle et en présence d'un composé phénolique comme co-catalyseur, ledit catalyseur de nickel étant présent à raison de q,5 à 5 % en poids dudit composé dioléfinique et ledit co-catalyseur phénolique étant présent à raison de 10 à 35 % en poids dudit composé dioléfinique.

Un premier but de la présente invention consiste à dimériser les diènes conjugués avec une vitesse nettement supérieure à celle connue jusqu'alors par le procédé décrit ci-dessus. Un autre but de la présente invention consiste, en ce qui concerne le butadiène-1,3, à le dimériser sélectivement en octatriène-1,3,6, octatriène-2,4,6 ou octatriène-1,3,7.

Pour atteindre les buts précités, le demandeur a mis au point un procédé de dimérisation de diènes conjugués en présence d'un catalyseur renfermant du nickel zérovalent et un composé de phosphore, caractérisé en ce que ledit catalyseur est obtenu par mise en présence de (A) au moins un complexe de nickel de formule NiZq dans laquelle q est le degré de coordination du nickel et Z est au moins un ligand capable de complexer le nickel et (B) au moins un ligand phosphoré de formule générale

$$L = R_1HN \left( \begin{array}{c} R_2 \\ | \\ C \\ | \\ R_3 \end{array} \right)_m OP(C_6H_5)_2$$

dans laquelle:

- $R_1$ est choisi parmi l'atome d'hydrogène et les radicaux hydrocarbonés,
- $R_2$ et $R_3$, identiques ou différents, sont choisis parmi l'atome d'hydrogène et les radicaux hydrocarbonés éventuellement porteurs d'au moins une fonction choisie parmi les fonctions alcool, thiol, thioether, amine, imine, acide, ester, amide et ether, et
- m est supérieur ou égal à 1.

Des exemples de radicaux $R_2$ ou $R_3$ sont notamment les radicaux méthyle, isopropyle, isobutyle, isoamyle, n-hydroxyéthyle, iso-hydroxyéthyle, n-aminobutyle, méthyléne-4 imidazolyle, N-n propylguanidyle, éthanoyle, acétamidoyle, n-propionyle, n-propionamidoyle, benzyle, p-hydroxybenzyle, méthyléne-3 indoxyle, méthanethioyle.

Ainsi la caractéristique du procédé selon l'invention réside dans la présence d'une quantité catalytique d'au moins un ligand L, chélate essentiellement monodenté de la famille des aminophosphinites. Les ligands de cette famille sont identifiables à l'aide de leurs spectres de résonance magnétique nucléaire du proton, du carbone 13 et du phosphore 31.

Le tableau I ci-après résume les données d'identification de certains aminophosphinites selon l'aminoalcool ou l'aminoacide dont ils sont issus:

TABLEAU I

| Aminoalcool ou Aminoacide | $^{31}\delta_P$ | $^{13}\delta_C$ | $^{1}\delta_H$ |
|---|---|---|---|
| Ephédrine | 112,3 | 127-132 (m)<br>84,5 (d)<br>61,1 (d)<br>31,8 (s)<br>15,2 (s) | 0,92-1,03 (d) (3H)<br>1,59 (s) (1H)<br>2,23 (m) (3H)<br>2,82 (m) (1H)<br>4,78-5,01 (m) (1H)<br>7,27 (m)(10H) |
| Valine | 113,7 | 127-135 (m)<br>69,2 (d)<br>65,9 (d)<br>34,5<br>29,3<br>18,9<br>18,8 | |
| Prolinol | 113,1 | | 1,69 (m) (5H)<br>2,85 (m) (2H)<br>3,20 (m) (1H)<br>3,64-3,87 (m) (2H)<br>7,4 (m)(10H) |
| Glycine | 113,5 | | 1,31 (s) (1H)<br>2,30 (s) (3H)<br>2,73 (t) (2H)<br>3,80-3,97(t-d)(2H)<br>7,14-7,48 (m)(10H) |

Dans ce tableau δ désigne le déplacement chimique exprimé en ppm par rapport à l'acide phosphorique (cas du phosphore 31) ou au tétraméthylsilane (cas du proton et du carbone 13). Les notations (m), (d) (t), (t-d) et (s) désignent respectivement un multiplet, un doublet, un triplet, un triplet dedoublé et un singulet.

Le ligand L utilisé dans le procédé selon l'invention peut être obtenu en faisant réagir dans un solvant hydrocarboné, à une température comprise entre -50°C et 110°C et sous atmosphère de gaz inerte un aminoalcool de formule générale

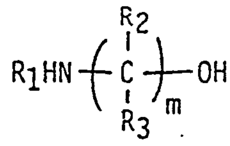 (I)

dans laquelle m, $R_1$, $R_2$ et $R_3$ ont les mêmes significations que précédemment, avec au moins un composé de formule $P(C_6H_5)_2$ Y dans laquelle Y est choisi parmi les atomes d'halogène et les radicaux amines, ledit composé étant dans un rapport molaire, relativement à l'aminoalcool, supérieur ou égal à 1.

Pour la synthèse des aminophosphinites, on préfère utiliser un composé dans lequel Y est un radical amine. Par solvant hydrocarboné on entend par exemple le benzène, le toluène. Pour faciliter l'élimination de l'acide chlorhydrique formé par la réaction, dans le cas où Y est un halogène, on peut avantageusement conduire celle-ci en présence d'un excès d'amine tertiaire, telle que par exemple la triéthylamine, qui précipitera sous forme de chlorhydrate. Le ligand phosphoré est isolé successivement par filtration du précipité suivie d'évaporation du solvant hydrocarboné sous vide. Il se présente généralement sous forme d'une huile visqueuse ou d'un solide à faible température de fusion. Les aminoalcools de formule (I) sont le plus souvent des produits soit commerciaux soit facilement accessibles par réduction d'aminoacides (ou de leurs esters méthyliques formylés). Lorsque le radical $R_2$ ou $R_3$ de l'aminoalcool de formule (I) est porteur d'une fonction, celle-ci peut être introduite par une réaction de fonctionnalisation bien connue; par exemple la fonction ester sera introduite par estérification de la fonction acide correspondante (cas des carbalcoxyhydroxyprolines). A titre d'exemples on peut citer le prolinol, l'hydroxyproline, les éphédrines et les aminoalcools N-méthylés dérivés des aminoacides naturels suivants: glycine, phénylglycine, phénylalanine, leucine, sérine, thréonine, histidine, lysine, arginine, isoleucine, valine, alanine, tyrosine, tryptophane, méthionine, cystéine, glutamine, asparagine, acide aspartique, acide glutamique, cystine.

Parmi les ligands Z couramment utilisables on peut citer le monoxyde de carbone, les halogènes, les oléfines, les diènes, le carbonate, les carboxylates et l'acétylacétone. Le degré de coordination q peut être couramment compris entre 2 et 4 inclusivement selon le ligand utilisé.

Lorsque le ligand Z est ionique (cas des halogènes, carboxylates, de l'acétylacétone et du carbonate), la réaction de dimérisation selon l'invention peut être avantageusement effectuée en présence de (C) au moins un activateur organométallique. L'activateur (C) peut être choisi parmi les dérivés aluminiques de formule $AlR_nX_{3-n}$, dans laquelle $1 \leqslant n \leqslant 3$, X est un atome d'halogène et R est un radical alkyle ayant de 1 à 12 atomes de carbone, les dérivés métalliques de formule MRp dans laquelle M est choisi parmi lithium, magnésium et zinc, R est un radical alkyle ou aryle ayant de 1 à 12 atomes de carbone et p est la valence du métal M, le borohydrure de sodium NaBH4 et l'hydrure de lithium et aluminium. Les constituants (A) et (C) sont généralement dans un rapport molaire (C)/(A) compris entre 0,01 et 10.

Parmi les diènes conjugués pouvant être soumis au procédé de dimérisation selon l'invention on peut citer notamment le butadiène-1,3, l'isoprène et le pipérylène. Le procédé selon l'invention peut être avantageusement mis en oeuvre à une température comprise entre 0° et 120°C, sous une pression comprise entre 1 et 15 bars pendant une durée comprise entre 2 et 400 minutes et le cas échéant en présence d'un solvant tel que, par exemple, le toluène. La proportion molaire de chaque composant (A) et (B) du catalyseur utilisé selon l'invention est avantageusement comprise entre 0,01 et % par rapport au butadiène-1,3.

En ce qui concerne le butadiène-1,3, lorsque la réaction de dimérisation est conduite en présence des composants (A), (B) et éventuellement (C), elle conduit avec une vitesse élevée et un rendement élevé, généralement supérieur ou égal à 80 %, à la formation sélective d'octa-triène-1,3,6 ou d'octatriène-2,4,6, selon la durée de la réaction. Toutefois on peut orienter préférentiellement la réaction de dimérisation vers la formation de méthylènevinylcyclopentane, ou d'octatriène -1,3,7 en la conduisant en présence en outre de (D) au moins un alcool aliphatique tel que, par exemple, le méthanol. Dans ce mode de réalisation de l'invention, le rapport molaire (D)/(A) peut être avantageusement compris entre 0,1 et 100.

Pour une meilleure compréhension de l'invention, il convient de noter que par mise en présence des composants (A) et (B) du catalyseur il se forme un complexe de nickel de formule $NiZ_{q-r}L_r$ dans laquelle Z, L et q ont les mêmes significations que précédemment et r est égal à 1 ou 2. Un tel complexe est identifiable à l'aide de son spectre de résonance magnétique nucléaire du phosphore 31.

Les exemples ci-après sont donnés à titre illustratif de la présente invention.

**Exemple 1** - Synthése d'un ligand L

On fait réagir dans le benzène sec sous atmosphère d'azote 1 mole de diméthylaminodiphénylphosphine avec 1 mole d'éphédrine (rendue anhydre par distillation azéotropique de l'eau par le benzène). Après dégagement de la diméthylamine, on évapore le benzène sous vide et l'on récupère 0,8 mole de phényl-1-(oxydiphénylphosphino)-1-(N-méthylamino)-2-méthyl-2-éthane de pureté chimique évaluée à 90 % (voir identification au tableau I).

**Exemple 2**

En opérant dans les mêmes conditions qu'à l'exemple 1, on remplace l'éphédrine par le prolinol (celui-ci peut être aisément obtenu par réduction de l'acide pyrrolidine-2 carboxylique (proline) au moyen de $LiAlH_4$ en suspension dans la tétrahydrofuranne). On récupère 0,8 mole d'une aminophosphinite dénommée NH PROLIPHOS de pureté chimique évaluée à 90 %.

**Exemple 3**

En opérant dans les mêmes conditions qu'à l'exemple 1, on remplace l'éphédrine par le glycinol (préparé à partir de la glycine par monoformylation de la fonction amine, estérification de la fonction acide, puis réduction par $LiAlH_4$. On récupère 0,8 mole d'une aminophosphinite dénommée NH GLYPHOS de pureté chimique évaluée à 90 %.

**Exemples 4 à 7**

Dans un tube en verre on introduit sous azote 2,5 millimole de ligand L et 2,5 millimole de bis (cyclooctadiène -1,5) nickel. Après refroidissement on ajoute une solution contenant 125 millimoles de butadiène -1,3 dans 10 $cm^3$ de toluène. On ferme le tube réactionnel et on le place dans un bain thermostaté à 40°C. L'agitation est assurée par un barreau aimanté mû magnétiquement. Après une durée de réaction t (exprimée en minutes), le contenu du tube est filtré sur silice. On récupère une solution limpide et incolore. On trouvera dans le tableau II ci-après, en fonction de la nature du ligand L et de la durée t utilisés dans chaque exemple, la valeur du taux de conversion C (exprimé en pourcent) ainsi que la valeur des sélectivités (exprimées en pourcent) en octatriène -1,3,6 (OCT-1), vinyl -4 cyclohexène (VCH), cyclooctadiène-1,5 (COD) et octatriène-2,4,6 (OCT-2), abstraction faite du cyclooctadiène -1,5 provenant de la décomposition du catalyseur.

**Tableau II**

| Exemple | L | t | C | OCT-1 | VCH | COD | OCT-2 |
|---|---|---|---|---|---|---|---|
| 4 | NH EPHOS | 10 | 90 | 95,8 | 3,2 | 1,0 | 0 |
| 5 | NH PROLIPHOS | 30 | 90 | 98,7 | 0,7 | 0,6 | 0 |
| 6 | NH GLYPHOS | 40 | 90 | 95,8 | 2,7 | 1,5 | 0 |
| 7 | NH EPHOS | 20 | 100 | 0 | 3,8 | 0,8 | 87,8 |

Dans l'exemple 7 les autres produits formés, soit 7,6 %, sont des isomères.

**Exemples 8 à 11**

On effectue la dimérisation du butadiéne-1,3 dans des conditions analogues à celles de l'exemple 4, c'est-à-dire en présence de NH EPHOS, aux deux exceptions suivantes près:

- la durée de réaction est portée à 60 minutes et la température de réaction est portée à 60°C.
- on introduit en outre du méthanol dans le tube réactionnel.

On trouvera dans le tableau III ci-après, en fonction du rapport molaire

$\frac{MeOH}{Ni}$,

les sélectivités (exprimées en pourcent) en octatriène -1,3,6 (OCT-6), octatriéne-1,3,7 (OCT-7), méthylènevinylcyclopentane (MVCP), vinyl-4 cyclohexène (VCH)et cyclooctadiène-1,5 (COD), abstraction faite du cyclooctadiène-1,5 provenant de la décomposition du catalyseur.

**Tableau III**

| Exemple | $\frac{MeOH}{Ni}$ | OCT-6 | OCT-7 | MVCP | VCH | COD |
|---|---|---|---|---|---|---|
| 8 | 0 | 88,6 | - | 3,1 | 2,6 | 5,7 |
| 9 | 2 | 38,6 | 9,4 | 13,5 | 17,7 | 20,8 |
| 10 | 12 | 27,1 | 21,9 | 39,5 | 4,2 | 6,3 |
| 11 | 36 | 3,2 | 23,9 | 60,3 | 9,4 | 3,2 |

**Exemples 12 et 13**

On effectue la dimérisation du butadiéne-1,3 dans des conditions analogues à celles de l'exemple 4, aux exceptions suivantes près:

- la température de réaction est portée à 60°C.
- les quantités, toujours équimolaires, de ligand L et de bis(cyclo-octadiène-1,5) nickel introduites dans le tube sont diminuées conformément au tableau IV ci-après (exprimées en millimoles).
- la durée de réaction t est augmentée conformément au tableau IV ci-après. On trouvera dans le tableau IV ci-après les sélectivités (exprimées en pourcent) en octatriène 1,3,6 (OCT-1), vinyl-4-cyclohexène (VCH) et cyclooctadiène-1,5 (COD), abstraction faite du cyclooctadiène-1,5 provenant de la décomposition du catalyseur.

Les autres produits formés, en proportion de l'ordre de 2 %, sont du méthylènevinylcyclopentane et des oligomères supérieurs.

**Tableau IV**

| Exemple | L | t | C | OCT-1 | VCH | COD |
|---|---|---|---|---|---|---|
| 12 | 0,0612 | 80 | 85 | 92,7 | 2,6 | 2,3 |
| 13 | 0,0250 | 360 | 60 | 92,8 | 2,7 | 2,5 |

**Exemples 14 et 15**

On effectue la dimérisation du butadiène-1,3 dans des conditions analogues à celles des exemples 4 et 5, c'est-à-dire en présence de NH EPHOS ou NH PROLIPHOS, aux deux exceptions suivantes près:
- la durée de réaction est portée à 360 minutes et la température de réaction est portée à 60°C.
- on introduit en outre du méthanol dans le tube réactionnel dans un rapport molaire

$$\frac{MeOH}{Ni}$$

égal à 50.

On trouvera dans le tableau V ci-après, en fonction de la nature du ligand L, les sélectivités (exprimées en pourcent) en octatriène-1,3,6 (OCT-6), octatriène-1,3,7 (OCT-7) et méthylènevinylcyclopentane (MVCP). Les autres produits formés sont des oligomères supérieurs.

**Tableau V**

| Exemple | L | OCT-6 | OCT-7 | MVCP |
|---|---|---|---|---|
| 14 | NH.EPHOS | 6,3 | 27,3 | 53,3 |
| 15 | NH PROLIPHOS | 13,3 | 74,2 | 7,0 |

**Exemples 16 et 17**

Dans un tube en verre on introduit sous azote 0,8 millimole d'acétylacétonate de nickal anhydre et 5 $cm^3$ de toluène. Dans cette solution on introduit à 0°C, sous atmosphère d'azote et de butadiène, une solution contenant 1,6 millimole de triéthylaluminium dans 5 $cm^3$ de toluène. On introduit ensuite à cette température 0,8 millimole de ligand NH EPHOS, 2 grammes d'heptane et 185 millimoles de butadiéne-1,3. On ferme le tube réactionnel et on le place dans un bain thermostaté agité à 40°C. Après une durée de réaction t (exprimée en minutes), le contenu du tube est traité comme dans l'exemple 4. On trouvera dans le tableau VI ci-après, en fonction de la durée t, la valeur du taux de conversion C ainsi que des sélectivités en octa-triène-1,3,6 (OCT-1), vinyl-4 cyclohexène (VCH), cyclooctadiène-1,5 (COD) et octratriène-2,4,6 (OCT-2). Le complement des produits formés à 100 % est constitué par d'autres dimères non identifiés.

**Exemple 18**

On effectue la dimérisation du butadiène-1,3 dans des conditions analogues à celles de l'exemple 16, à l'exception suivante: on remplace la solution de 1,6 millimole de triéthylaluminium dans 5 $cm^3$ de toluène par une solution de 1,6 millimole d'hydrure double de lithium et aluminium dans 2 $cm^3$ de tétrahydrofurane anhydre. Les résultats expérimentaux figurent au tableau VI.

Tableau VI

| Exemple | t | C | OCT-1 | VCH | COD | OCT-2 |
|---|---|---|---|---|---|---|
| 16 | 30 | 75 | 96,4 | 1,6 | 0,9 | 0 |
| 17 | 165 | 100 | 0 | 1,8 | 1,6 | 90,2 |
| 18 | 60 | 80 | 95,0 | 1,8 | 1,3 | 0,8 |

**Exemple 19**

Dans un tube en verre on introduit sous azote 2,5 millimole du ligand préparé à l'exemple 1 et 2,5 millimole de bis (cyclooctadiène-1,5) nickel. On ajoute une solution contenant 125 millimoles de pentadiène-1,3 (mélange à 28 % de l'isomère cis et 72 % de l'isomère trans) dans 10 $cm^3$ de toluène. On ferme le tube réactionnel et on le place dans un bain thermostaté à 40°C, l'agitation étant assurée magnétiquement. Après une durée de réaction de 45 minutes, le contenu du tube est filtré sur silice. On récupère une solution limpide et incolore. Le taux de conversion est égal à 75 %. L'analyse des produits formés révéle des sélectivités de 70% en diméthyl-4,5-octatriène (isomère 1,3 trans, 6 trans) et de 21 % en diméthyl -4,5- octatriène (isomère 1,3 cis, 6 trans). Les autres isomères formés, soit 9%, n'ont pu être identifiés en raison de la proportion trop faible de chacun d'eux.

**Exemple 20**

Dans un tube en verre on introduit sous azote 2,9 millimoles d'acétylacétonate de nickel anhydre et 5 $cm^3$ de toluène. Dans cette solution on introduit une solution contenant 5,8 millimoles de triéthylaluminium dans $cm^3$ de toluène. On introduit ensuite 2,9 millimoles du ligand préparé à l'exemple 1 et 250 millimoles de pentadiène-1,3 (mélange à 28 % de l'isomère cis et 72 % de l'isomère trans). On ferme le tube réactionnel et on le place dans un bain thermostaté agité à 40°C. Après une durée de réaction de 240 minutes, le contenu du tube est traité comme dans l'exemple 2. Le taux de conversion est égal à 85 %. L'analyse des produits formés révèle des sélectivités de 51 % en diméthyl-4,5-octatriène(isomère 1,3 trans, 6 trans) et de 35 % en diméthyl-4,5-octatriène (isomère 1,3 cis, 6 trans). Les autres isomères formés, soit 14 %, n'ont pu être identifiés en raison de la proportion trop faible de chacun d'eux.

**Exemple 21**

Dans un autoclave en acier inoxydable on introduit sous flux d'azote une solution contenant 2,5 millimoles du ligand de l'exemple 1 et 2,5 millimoles de bis(cyclooctadiène-1,5) nickel dans 10 $cm^3$ de toluène. On ajoute ensuite 125 millimoles d'isoprène. Le réacteur est fermé et placé sur un agitateur magnétique, l'agitation étant assurée par un barreau aimanté. Une double enveloppe placée au tour de l'autoclave permet de stabiliser la température réactionnelle à l'aide d'un bain thermostaté fixé à 60°C. Après une durée de réaction de 360 minutes, le contenu du réacteur est filtré sur silice. On récupère une solution limpide et incolore. Le taux de conversion est égal à 94 %. L'analyse des produits formés révèle les sélectivités suivantes:

limonène: 5,5 %
diméthyl-2,4-vinyl-4 cyclohexène et
diméthyl-1,4-vinyl-4 cyclohexène: 12,5 %
diméthyl-1,4-cyclooctadiène-1,5 et
diméthyl-1,5-cyclooctadiène-1,5: 20,5 %
diméthyl-2,7-octatriène-1,3,6: 3,5 %
diméthyl-2,7-octatriène-2,4,6: 58 %

**Revendications**

1. Procedé de dimérisation d'un diène conjugué en présence d'un catalyseur renfermant du nickel zérovalent et un composé de phosphore, caractérisé en ce que ledit catalyseur est obtenu par mise en présence de (A) au moins un complexe de nickel de formule NiZq dans laquelle q est le degré de coordination du nickel et Z est au moins un ligand capable de complexer le nickel et (B) au moins un ligand phosphoré de formule générale

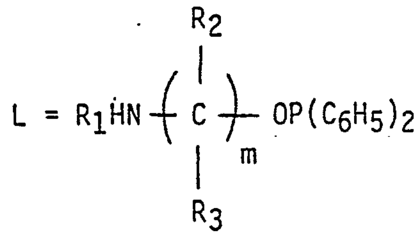

dans laquelle:
- $R_1$ est choisi parmi l'atome d'hydrogène et les radicaux hydrocarbonés,
- $R_2$ et $R_3$ sont choisis parmi l'atome d'hydrogène et les radicaux hydrocarbonés éventuellement porteurs d'au moins une fonction choisie parmi les fonctions alcool, thiol, thioether, amine, imine, acide, ester, amide et ether, et
- m est supérieur ou égal à 1.

2. Procédé selon revendication 1 caractérisé en ce que Z est choisi parmi le monoxyde de carbone, les halogènes, les diènes, le carbonate, les carboxylates et l'acétylacétone.

3. Procédé selon l'une des revendications 1 et 2, caractérisé en ce que q est compris entre 2 et 4 inclusivement.

4. Procédé selon l'une des revendications 1 à 3, le ligand Z étant ionique, caractérisé en ce que la dimérisation est effectuée en présence de (C) au moins un activateur organométallique.

5. Procédé selon la revendication 4, caractérisé en ce que le rapport molaire (C)/(A) est compris entre 0,01 et 10.

6. Procédé selon l'une des revendications 1 à 5, caractérisé en ce que la dimérisation est effectuée à une température comprise entre 0° et 120° C.

7. Procédé selon l'une des revendications 1 à 6, caractérisé en ce que la dimérisation est effectuée sous une pression comprise entre 1 et 15 bars.

8. Procédé selon l'une des revendications 1 à 7, caractérisé en ce que la dimérisation est effectuée pendant une durée comprise entre 2 et 400 minutes.

9. Procédé selon l'une des revendications 1 à 8, caractérisé en ce que la dimérisation est effectuée en présence d'un solvant.

10. Procédé selon l'une des revendications 1 à 9, caractérisé en ce que la proportion molaire de chaque composant (A) et (B) du catalyseur est comprise entre 0,01 et 3 % par rapport au diène conjugué.

11. Procédé selon l'une des revendications 1 à 10, le diène conjugué étant le butadiène-1,3, caractérisé en ce que la dimérisation est effectuée en présence de (D) au moins un alcool aliphatique.

12. Procedé selon la revendication 11, caractérisé en ce que le rapport molaire (D)/(A) est compris entre 0,1 et 100.

**Patentansprüche**

1. Verfahren zur Dimerisation eines konjugierten Diens in Gegenwart eines Katalysators, der nullwertiges Nickel und eine Phosphorverbindung enthält, dadurch gekennzeichnet, daß der genannte Katalysator dargestellt wird durch Zusammenbfingen von (A) mindestens einem Nickelkomplex der Formel NiZq, in der q die Koordinationszahl des Nickels und Z mindestens ein zur Komplexbildung des Nickels geeigneter Ligand ist, und (B) mindestens einem phosphorhaltigen Liganden der allgemeinen Formel

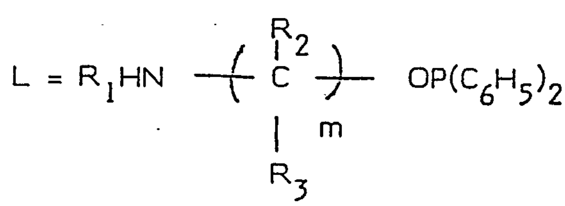

in der:

- $R_1$ ausgewählt ist aus der Gruppe bestehend aus dem Wasserstoffatom und den Kohlenwasserstoff-Radikalen,

- $R_2$ und $R_3$ ausgewählt sind aus der Gruppe bestehend aus dem Wasserstoffatom und den Kohlenwasserstoff-Radikalen, wobei letztere gegebenenfalls Träger mindestens einer Funktion sind, die ausgewählt ist aus den Alkohoi-, Thiol-, Thioether-, Amin-, Imin-, Säure-, Ester-, Amid- und Ätherfunktionen, und

- m größer als oder gleich 1 ist.

2. Verfahren nach Anspruch 1, dadurch gekennzeichnet, daß Z ausgewählt ist aus der Gruppe bestehend aus Kohlenmonoxid, den Halogenen, den Dienen, dem Carbonat, den Carboxylaten, und dem Acetylaceton.

3. Verfahren nach Anspruch 1 oder 2, dadurch gekennzeichnet, daß q 2 bis 4 ist.

4. Verfahren nach einem der Ansprüche 1 bis 3, bei dem der Ligand Z ionisch ist, dadurch gekennzeichnet, daß die Dimerisation in Gegenwart von (C) mindestens einem organometallischen Aktivator durchgeführt wird.

5. Verfahren nach Anspruch 4, dadurch gekennzeichnet, daß das Molverhaltnis (C)/(A) zwischen 0,01 und 10 beträgt.

6. Verfahren nach einem der Ansprüche 1 bis 5, dadurch gekennzeichnet, daß die Dimerisation bei einer Temperatur zwischen 0° und 120°C durchgeführt wird.

7. Verfahfen nach einem der Ansprüche 1 bis 6, dadurch gekennzeichnet, daß die Dimerisation unter einem Druck zwischen 1 und 15 bar durchgeführt wird.

8. Verfahren nach einem der Ansprüche 1 bis 7, dadurch gekennzeichnet, daß die Dimerisation während einer Dauer zwischen 2 und 400 min durchgeführt wird.

9. Verfahren nach einem der Ansprüche 1 bis 8, dadurch gekennzeichnet, daß die Dimerisation in Gegenwart eines Lösungsmittels durchgeführt wird.

10. Verfahren nach einem der Ansprüche 1 bis 9, dadurch gekennzeichnet, daß der Molanteil jedes Bestandteils (A) und (B) des Katalysatofs zwischen 0,01 und 3%, bezogen auf das konjugiefte Dien, beträgt.

11. Verfahren nach einem der Ansprüche 1 bis I0, bei dem das konjugierte Dien Butadien-1,3 ist, dadurch gekennzeichnet, daß die Dimerisation in Gegenwart von (D) mindestens einem aliphatischen Alkohol durchgeführt wird.

12. Verfahren nach Anspruch 1 1, dadurch gekennzeichnet, daß das Molverhältnis (D)/(A) zwischen 0,1 und 100 beträgt.

**Claims**

1. Process for dimerization of a conjugated diene in the presence of a catalyst containing zerovalent nickel and a phosphorus compound, characterized in that the said catalyst is obtained by bringing together (A) at least one nickel complex of formula $NiZ_q$ in which q is the coordination number of the nickel and Z is at least one ligand capable of complexing nickel and (B) at least one phosphorus-containing ligand of general formula

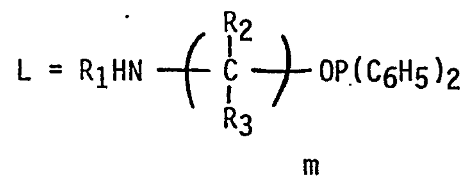

in which:

- $R_1$ is chosen from a hydrogen atom and hydrocarbon radicals,

- $R_2$ and $R_3$, which may be identical or different, are chosen from a hydrogen atom and hydrocarbon radicals optionally bearing at least one group chosen from alcohol, thiol, thioether, amine, imine, acid, ester, amide and ether groups, and

- m is greater than or equal to 1.

2. Process according to claim 1, characterized in that Z is chosen from carbon monoxide, halogens, dienes, carbonate, carboxylates and acetylacetone.

3. Process according to one of claims 1 and 2, characterized in that q is between 2 and 4 inclusive.

4. Process according to one of claims 1 to 3, the ligand Z being ionic, characterized in that the dimerization is performed in the presence of (C) at least one organometallic activator.

5. Process according to claim 4, characterized in that the mole ratio (C)/(A) is between 0.01 and 10.

6. Process according to one of claims 1 to 5, characterized in that the dimerization is performed at a temperature between 0° and 120°C.

7. Process according to one of claims 1 to 6, characterized in that the dimerization is performed under a pressure of between 1 and 15 bars.

8. Process according to one of claims 1 to 7, characterized in that the dimerization is performed for a time between 2 and 400 minutes.

9. Process according to one ot claims 1 to b, characterized in that the dimerization is performed in the presence of a solvent.

10. Process according to one of claims 1 to 9, characterized in that the molar proportion of each component (A) and (B) of the catalyst is between 0.01 and 3 % relative to the conjugated diene.

11. Process according to one of claims 1 to 10, the conjugated diene being 1,3-butadiene, characterized in that the dimerization is performed in the presence of (D) at least one aliphatic alcohol.

12. Process according to claim 11, characterized in that the mole ratio (D)/(A) is between 0.1 and 100.